# EUROPEAN PATENT APPLICATION

(11) **EP 4 631 551 A1**
(43) Date of publication of application: **15.10.2025**
(21) Application number: 24168929.8
(22) Date of filing: 08.04.2024
(51) Int. Cl.: A61M 11/00, A61M 15/08, B05B 11/10

(54) **MANUAL PUMP**

(71) Applicant: ALPLA Werke Alwin Lehner GmbH & Co KG, 6971 Hard (AT)
(72) Inventor: SHARMA, Sunil Kumar, 110092 Dehli (IN); LEE, Jaehwan, Hwaseong-si, Gyeonggi-do, Namyang-eup 18255 (KR)
(74) Representative: Kiwit, Benedikt

(57) **Abstract**

The invention relates to a manual pump (1) for a fluid dispenser, such a fluid dispenser for dispensing a fluid for intranasal administration, the pump (1) comprising: a pump body (2) delimiting a pump chamber (3) and having an inlet (21) connectable to a reservoir (4); a piston assembly (5) comprising a piston (6) and a piston rod (7), wherein the piston assembly (5) is arranged to move axially downwards to set a dose of fluid in the pump chamber (3) under pressure and arranged to move axially upwards to suck a fluid from the reservoir (4) into the pump chamber (3) via the inlet (21); and a valve (8) arranged to prevent a flow of the fluid from the pump chamber (3) into the inlet (21) as the piston assembly (5) moves axially downwards and to allow a flow of the fluid from the inlet (21) into the pump chamber (3) as the piston assembly (5) moves axially upwards; wherein:
a) the piston assembly (5) comprises a closing section (9) attached to the piston rod (7), and the valve (8) comprises an axially moveable valve body (81) with an opening (82) for conducting a fluid from the inlet (21) into the pump chamber (3), wherein the closing section (9) and the valve body (81) are arranged such that
- the opening (82) is closed by the closing section (9) as the piston rod (7) moves axially downwards, so that a flow of fluid from the pump chamber (3) into the inlet (21) is prevented, and
- the opening (82) is distanced from the closing section (9) as the piston rod (7) moves axially upwards, so that a flow of fluid from the inlet (21) into the pump chamber (3) is allowed; and/or

b) the pump (1) comprises one or more resilient elements (10, 11) arranged to interact with the piston assembly (5) and integrally formed with the piston (6) and/or piston rod (7).

## Description

### 1. Field of the invention

The present invention relates to a manual pump for a fluid dispenser, such as a fluid dispenser for dispensing a fluid for intranasal administration, a container comprising such a pump, and a method for manually pumping a fluid.

### 2. Technical background

Fluid dispensers are used in various applications, such as for administering a fluid, e.g. in the form of an aerosol, into a human's nose. However, such fluid dispensers are not easily recyclable. This is in particular because several components (springs, valve elements (e.g., a ball-shaped element), etc.) of the fluid dispenser require a certain material, e.g. metal, in order to function properly, whereas other components (container body, cap, etc.) are not restricted to a certain material and thus are for economic reasons (costs, weight, energy, etc.) made of a different material, e.g. plastic. Thus, a material mix, e.g. a mix of a metal and a plastic material, is present in the fluid dispenser, which renders the fluid dispenser not very recyclable.

Therefore, it is an object of the present invention to overcome one or more of the aforementioned drawbacks, in particular to increase recyclability while reducing, or at least not increasing, costs.

These and other objects, which become apparent upon reading the following description, are solved by the subject-matter of the independent claims. The dependent claims refer to preferred embodiments of the invention.

### 3. Summary of the invention

According to a first aspect of the invention, a manual pump for a fluid dispenser, such as a fluid dispenser for dispensing a fluid, e.g. in the form of an aerosol, for intranasal administration, is provided. The pump comprises: a pump body delimiting a pump chamber and having an inlet connectable to a reservoir; a piston assembly comprising a piston and a piston rod, wherein the piston assembly is arranged to move axially downwards to set a dose of fluid in the pump chamber under pressure and arranged to move axially upwards to suck a fluid from the reservoir into the pump chamber via the inlet; and a valve arranged to prevent a flow of the fluid from the pump chamber into the inlet as the piston assembly moves axially downwards and to allow a flow of the fluid from the inlet into the pump chamber as the piston assembly moves axially upwards.

According to alternative a), the piston assembly comprises a closing section attached to the piston rod, and the valve comprises an axially moveable valve body with an opening for conducting a fluid from the inlet into the pump chamber, wherein the closing section and the valve body are arranged such that: the opening is closed by the closing section as the piston rod moves axially downwards, so that a flow of fluid from the pump chamber into the inlet is prevented, and the opening is distanced from the closing section as the piston rod moves axially upwards, so that a flow of fluid from the inlet into the pump chamber is allowed.

This achieves in particular the advantage that the valve body is not required to be made of a specific material, such as metal. Thus, the valve and/or valve body can be (e.g., entirely) made of a material, such as a plastic material, that increases the recyclability and at the same time reduces costs, e.g. costs due to production or transporting the pump. In particular, the proposed manual pump opens the possibility to use only one type of material (such as a plastic material, e.g. PP) for the entire pump (to provide a so-called mono material pump). This is in particular not possible in manual pumps, in which for proper functionality a check valve with a closure element made of metal (such as stainless steel) or any other material with a density greater than 1 g/cm³ (such as POM or glass) is required. Thereby, the manual pump is not only advantageous with respect to recyclability but also low-cost. As such, the manual pump is in particular suitable to replace a container (such as a bottle) that is squeezable to dispense a fluid.

According to alternative b), which is provided in addition to, or instead of, alternative a), the pump comprises one or more resilient elements arranged to interact with the piston assembly, wherein the one or more resilient elements are integrally formed with the piston and/or piston rod.

This achieves in particular the advantage that the one or more resilient elements and the piston and/or piston rod can be produced with a reduced number of different types of materials and with an easier provision, in particular with a reduced number of production and handling steps and with reduced production energy. Thus, recyclability is increased and costs are reduced.

In an embodiment, the valve body can be connected via a connection to the piston rod such that when the piston rod moves axially upwards the valve body moves axially upwards as well. Thus, bringing the valve body back into its initial upper position, in which the valve body comes into contact or is hit by the closing section moving axially downward, is simplified.

The connection may comprise a first engagement section attached, directly or indirectly, to the piston rod and a second engagement section attached to, preferably integrally formed with, the valve body. The first and second engagement sections may be arranged to engage with one another as the piston rod moves axially upwards, so that the piston rod carries the valve body axially upwards during at least part of the piston rod's movement axially upwards.

The first engagement section may extend radially, such as radially inwards. The second engagement section may extend radially, such as radially outwards.

Each of the first engagement section and/or the second engagement section may be provided by a respective protrusion, such as a snapping protrusion.

The valve body may be connected to the piston rod with axial play, wherein the axial play is preferably in a range of 0.1-1mm. The axial play may be defined by the first and/or second engagement sections.

At least one of the first and second engagement sections may delimit a passage that allows the flow of fluid from the valve body's opening into the pump chamber, when the opening is not closed by the closing section.

An axial wall may be attached to the piston rod, wherein the first engagement section is attached to the axial wall. Additionally or alternatively, a hood, such as in the form of a (e.g., slitted) cage, may be attached to the piston rod, wherein the first engagement section is attached to the hood. The hood may comprise the axial wall.

The valve body may be axially guided by the pump body, such as by a duct of the pump body. The duct preferably comprises the inlet of the pump body.

The valve body may be connected to the pump body by a frictional connection. By the frictional fit, or by any other connection, the valve body may be axially moveable with a mechanical resistance. Thus, the closing section moving axially downwards and against the valve body can effectively close, in particular seal, the opening of the latter.

The pump body may comprise a stopper, such as in the form of a protrusion or a bridge, arranged to limit a movement of the valve body along an axial downward direction. This simplifies assembly of the pump. For example, the valve body may rest, or may be supported, on the stopper and then the piston assembly may be connected, e.g. by a snap fit or a snap action, to the valve body resting on the stopper, so that, for example, the valve body is connected to the piston rod with axial play.

The stopper may be further arranged to limit a movement of a (dip) tube, which can be connected to the pump body, along an axial upward direction.

The stopper may be arranged in the duct.

The pump body may comprise fastening means, such as a thread and/or snap elements, for connecting the pump to a container body.

The piston may comprise an outlet valve section configured to move between an open position that allows a fluid flow from the pump chamber into an outlet channel and a closed position that prevents a fluid flow from the pump chamber into the outlet channel, wherein the outlet valve section is configured to move into the open position when a pressure inside the pump chamber exceeds a defined pressure. Thus, the outlet valve section in particular ensures that a fluid flows from the pump chamber into the outlet channel only if the fluid has a pressure that is high enough.

At least part of the outlet channel may be provided in the piston rod.

The outlet valve section may be comprise wall, such as a top wall, of the piston and/or may be arranged to delimit the pump chamber such that a pressure inside of the pump chamber directly acts on the outlet valve section.

When the opening is closed by the closing section, a seal may be provided between the closing section and the valve body, wherein the seal is preferably adapted to withstand a pressure at least equal to the defined pressure. The seal may be provided by the closing section being pressed against the valve body during the closing section's movement axially downwards. The seal may be provided with or without a sealing element attached to the closing section and/or the valve body.

The outlet valve section may be biased, e.g. by a resilient element such as a spring, into the closed position. The resilient element may be arranged between the outlet valve section and a section attached to and preferably integrally formed with the piston rod.

The piston may comprises a hollow body that delimits the pump chamber. The piston may comprise one or more walls, such as an (e.g., radially) extending top wall and an (e.g., axially) extending sidewall. The top wall may comprise at least part of the outlet valve section.

The piston may comprises a sealing element, such as a sealing lip, arranged to contact the pump body, in particular an inner side of the pump body. Thus, the sealing element may help that a pressure can build up in the pump chamber as the pressure assembly moves axially downwards. The sealing element may be attached to the sidewall of the piston.

The piston assembly may comprise a piston connector, such as a in the form of a plug, attached, preferably by a snap fit, to the piston rod and comprising the closing section.

The piston plug may extend through the piston and/or may be coaxial with the piston.

At least part of the piston connector may be arranged or received in the piston rod.

The pump may comprise an outlet channel that is at least partly arranged, or at least partly extends, in the piston connector. The outlet channel may extend both in the piston connector and in the piston rod and/or may terminate in a dispensing opening, e.g. provided at the tip of the piston rod. Via the dispensing opening the fluid may be dispensed from the pump, e.g. in an aerosolized form. Preferably, the dispensing opening comprises an aerosolizer, such as a swirl aerosolizer, for aerosolizing the fluid.

The piston connector preferably comprises an inlet through which a fluid can flow from the outlet valve section into the outlet channel. In other words, when the outlet valve section is in the open position, a fluid can flow via the piston connector's inlet into the outlet channel. For example, when the outlet valve section is in the open position, a space is provided between (an inner section of) the piston and (an outer section of) the piston connector, wherein a fluid can flow from the pump chamber into said space and then from this space via the inlet into the outlet channel.

The outlet valve section and/or the piston may be axially moveable relative to the piston connector. In particular, when a pressure inside of the pump chamber exceeds a defined pressure, the outlet valve section and/or the piston may be axially moved upwards relative to the piston connector, thereby, for example, allowing a flow of fluid from the pump chamber into the outlet channel.

At least part of the piston connector may be arranged in a space delimited by the piston and/or in the pump chamber.

The piston assembly, such as the piston connector, may comprise an axial, preferably central protrusion extending axially and into the opening of the valve body, e.g. for axially guiding the valve body.

The one or more resilient elements may comprise a return, or main, resilient element arranged to move at least the piston rod axially upwards when an actuation pressure to move the piston rod axially downwards is removed. The return resilient element may move or force the piston rod and thus the piston axially upwards, whereby the pump chamber expands and thus, a pressure in the pump chamber is reduced. As during this upwards movement the opening of the valve body is not closed by the closing element, the reduced pressure may suck a fluid from the reservoir via the valve body into the pump chamber.

The return resilient element may be integrally formed with the piston rod.

The one or more resilient elements may comprise a valve (or pre-compression or secondary) resilient element to bias (or pre-compress or pre-tension) an outlet valve section, e.g. that described above, into a closed position.

The valve resilient element may be integrally formed with the piston, e.g. with the outlet valve section and/or the top wall of the piston.

The one or more resilient elements and preferably the piston assembly may be arranged coaxially.

At least one, or each, of the one or more resilient elements may be a spring. The spring may comprise two spring helixes that are connected with one another via one or more bridges. With such a spring design, not only load on the coils of the spring can be reduced but also too much expanding (in a radial direction) can be avoided when compressed.

For example, the spring may comprise a top plate, such as top ring plate having a hole formed at the center thereof, first and second spring members and a pair of connection spring members. The first spring member is erectly extended downward from one side undersurface of the top plate while rotating in a clockwise direction. The second spring member is erectly extended downward from the other side undersurface of the top plate which is a position symmetrical to one side undersurface thereof, while rotating in the same direction as the first spring member. The pair of connection spring members is erectly extended downward from two center surfaces between one side undersurface and the other side undersurface of the top plate, while rotating in the opposite direction (in a counterclockwise direction) to the first and second spring members, in such a manner as to be connected alternately to tops and undersides of the first spring member and the second spring member. For example, the spring may be according to the cylindrical spring (260) in EP 3 854 486 B1.

The pump may comprise an attachment section for attaching the piston assembly to the pump body, e.g. by a snap fit.

The attachment section is preferably attached to the pump body so as to remain stationary as the piston (assembly) moves axially.

The attachment section may be integrally formed with the piston assembly, such as with the piston rod.

At least one of the resilient elements, such as the return resilient element, may be integrally formed with the attachment section.

The attachment section may be annular. The attachment section may comprise a plate, such as a ring plate, wherein one or more (e.g., annular) attachment sub-sections, by which the attachment section can be attached to the pump body, may extend (e.g., axially, or transversely, such as perpendicularly, with respect to the plate) from the plate. The one or more attachment sub-sections may comprise one or more snap elements.

The pump body may comprise an (pump body) opening that can be (fluidly) connected to a reservoir of a container body, wherein the pump body (and preferably the attachment section) delimits at least part of a flow path that has a first end connected to this opening and a second end connected to an outside, wherein the piston is configured to move from an upper position, in which the flow path is blocked by the piston, preferably by a wedge-shaped element of the piston, axially downwards into a lower position, in which the flow path is not blocked by the piston, so that a gas, such as air, can flow from the outside into the inside of the container body. Thus, during at least part of the motion of the piston (assembly) axially upwards from the lower position to the upper position, a gas, such as air, can flow via the flow path into the reservoir to replace the space from the liquid drawn from the reservoir.

When the flow path is blocked by the piston, the piston may contact at least the pump body, in particular an inner side thereof. In particular, a section of the flow path may be delimited on one side by the pump body and on another side by an element attached to the pump body, e.g. by the attachment section, wherein the piston, such as the wedge-shaped element, is inserted into this section of the flow path to block it.

The wedge-shaped element may extend axially upwards and/or may be connected to a sidewall of the piston.

The pump may comprise one or more ribs, which preferably extend radially inward, that delimit the flow path. The one or more ribs may rest on, or contact, the piston, such as on an outer side of the piston. The attachment section may comprise the one or more ribs.

At least part of the flow path may extend between a section of the pump body and a section of the attachment section, such as a section provided by one or more attachment sub-sections.

Preferably, the valve body and/or the one or more resilient elements is/are made of a plastic material, such as polypropylene (PP). The valve body and/or the one or more resilient elements may be made of a material with a density lower than 1 gr/cm3 and/or a density lower than that of the fluid to be pumped or contained in the container body.

In an embodiment, the pump body, the piston assembly and the valve body are all made of the same material, such as a plastic material, in particular PP.

In an embodiment, the pump body, the piston assembly and the one or more springs are all made of the same material, such as a plastic material, in particular PP.

In an embodiment, the entire pump is made of only one type of material, such as a plastic material, in particular PP.

The valve body may have a tubular shape and/or may be a sleeve.

The pump may comprise a cap arranged to cover a dispensing opening of the pump, wherein the dispensing opening may be provided in the piston rod. The cap may comprise an opening that fluidly connects the dispensing opening with an outside or environment of the pump.

The pump may comprise a manually operable actuator arranged to enclose at least part of the piston assembly and at least part of the pump body, wherein the actuator is axially moveable relative to the pump body. The actuator may be arranged such that at least part of the flow path (see above) is arranged between a section of the actuator and a section of the pump body. The actuator may be stepped for easy user actuation. The actuator may be attached to the piston rod, e.g. by a snap fit. The actuator may comprise an opening, e.g. at its top, that fluidly connects to a dispensing opening of the piston rod and/or the outlet channel.

The pump may comprise a seal arranged on the pump body, e.g. on an underside thereof, and arranged to contact a container body to be used with the hand pump. Thus, when the pump body is attached to the container body, the seal is clamped between the pump body and the container body, e.g. a neck thereof. The seal may be a gasket or a sealing ring.

The pump may be void of any (integrated) measures taken to prevent microbial contamination when in use. This simplifies the design of the pump.

The pump may have dimensions such that it can be easily held by a human's hand. For example, the pump may have a height (parallel to the axial direction) in a range of 50-80mm, preferably 60-70mm, and/or a width (perpendicular to the axial direction) in a range of 20-35mm, preferably 25-30mm.

According to a second aspect of the invention, a container comprising a container body, such as a bottle, and a manual pump as described above is provided.

The container may comprise a (dip) tube connected to the inlet of the manual pump and extending into (a reservoir of) the container body. The tube may be inserted into the pump body, such as into the duct that may receive the valve body. By the stopper, an axial insertion of the tube into the pump body may be limited. This achieves the advantage of an easy assembly. For example, the stopper may comprise a front side and a back side, wherein the valve body may contact the front site and the tube may contact the back side.

The container body may contain (in its reservoir) a fluid, such as a formulation, e.g. in the form of a liquid. To prevent microbial contamination when in use, the fluid may contain preservatives, such as benzalkonium chloride (BAC).

The container may have dimensions such that it can be easily held by a human's hand. For example, the container may have a height (parallel to the axial direction) in a range of 90-120mm, preferably 100-110mm, and/or a width (perpendicular to the axial direction) in a range of 20-35mm, preferably 25-30mm.

According to a third aspect of the invention, a method for manually pumping a fluid from a reservoir, e.g. by using a manual pump as described above, is provided. The method comprises the steps of: moving, in a down stroke, a piston assembly comprising a piston and a piston rod axially downwards to set a dose of fluid in a pump chamber under pressure, wherein during at least part of the down stroke an opening of a valve body is closed by a closing section attached to the piston rod so that a flow of fluid from the pump chamber via the opening into an inlet connected to the valve body is prevented; and moving, in an up stroke, the piston assembly axially upwards, wherein during at least part of the up stroke the opening is distanced from the closing section so that a flow of fluid from the inlet via the opening into the pump chamber is allowed.

### 4. Description of a preferred embodiment

In the following, the invention is described exemplarily with reference to the enclosed figures, in which
- Figure 1: is a schematic lateral side view of a manual pump according to an embodiment, wherein the manual pump is connected to a container body;
- Figure 2: is a schematic cross-sectional side view of the manual pump shown in figure 1;
- Figure 2A: is a schematic perspective detail showing the dispensing opening;
- Figure 3: is a schematic detail of figure 2;
- Figure 4: is a schematic detail of figure 2;
- Figure 5: is a schematic detail of certain components shown in figure 2 ("transport position" during an up stroke);
- Figure 6: is a schematic detail of a piston connector and valve body according to the embodiment;
- Figure 7: is a schematic perspective view of the piston rod and the return resilient element according to the embodiment;
- Figure 8: is a schematic perspective view of the piston and the valve resilient element according to the embodiment;
- Figure 9: is a schematic perspective view of the valve body according to the embodiment;
- Figure 10: is a schematic perspective view of the piston connector according to the embodiment;
- Figure 11: is a schematic perspective view of the pump body according to the embodiment;
- Figure 12: is a schematic perspective view of the cap according to the embodiment;
- Figure 13: is a schematic perspective view of the actuator according to the embodiment;
- Figure 14: is a schematic cross-sectional side view of the manual pump according to the embodiment in an initial (upper) position;
- Figure 15: is a schematic cross-sectional side view of the manual pump according to the embodiment in a down stroke position;
- Figure 16: is a schematic cross-sectional side view of the outlet valve section of the manual pump according to the embodiment in a first phase during the down stroke;
- Figure 17: is a schematic cross-sectional side view of the outlet valve section of the manual pump according to the embodiment in a second phase during the down stroke;
- Figure 18: is a schematic cross-sectional side view of the manual pump according to the embodiment in an end (upper) position;
- Figure 19: is a schematic cross-sectional side view of the manual pump according to the embodiment in an up stroke position.

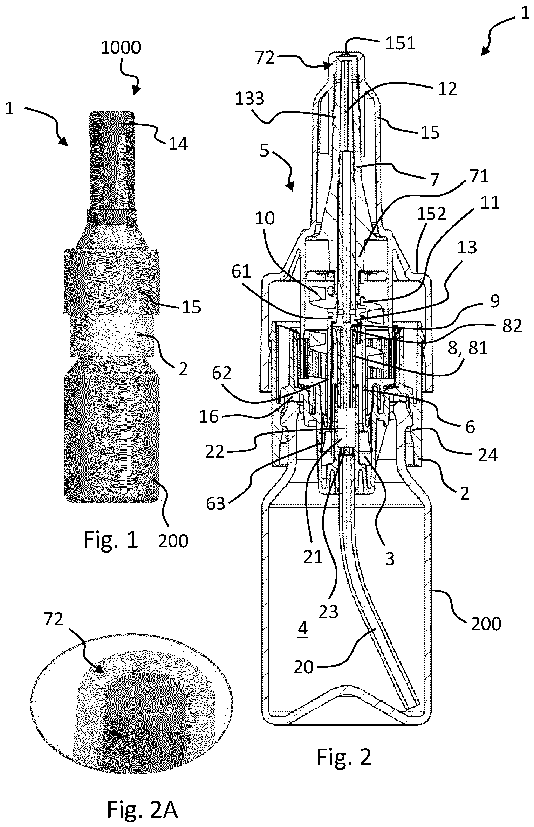

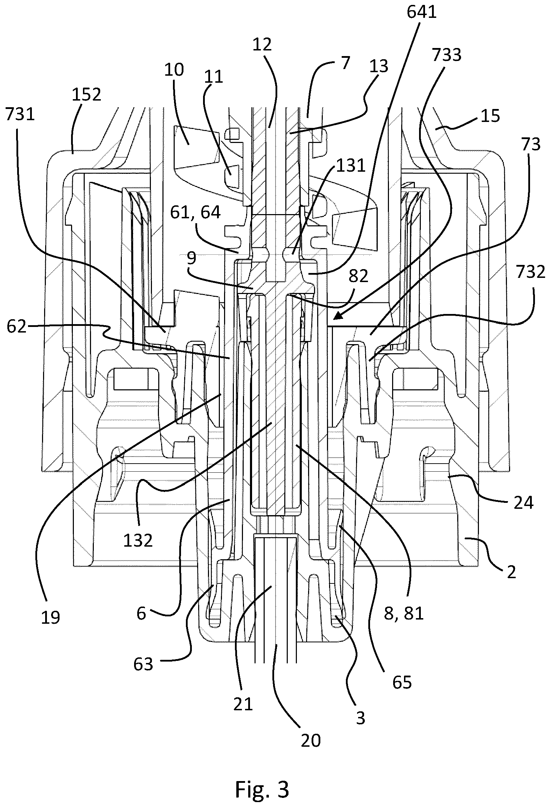

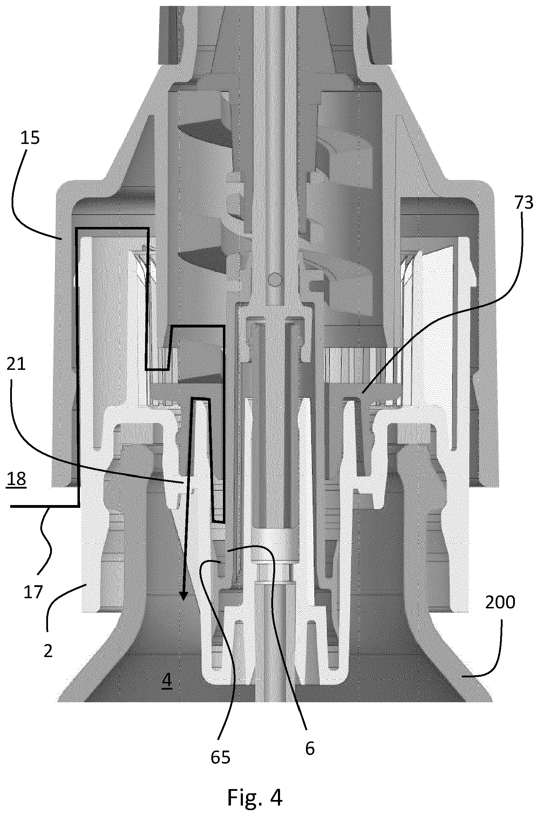

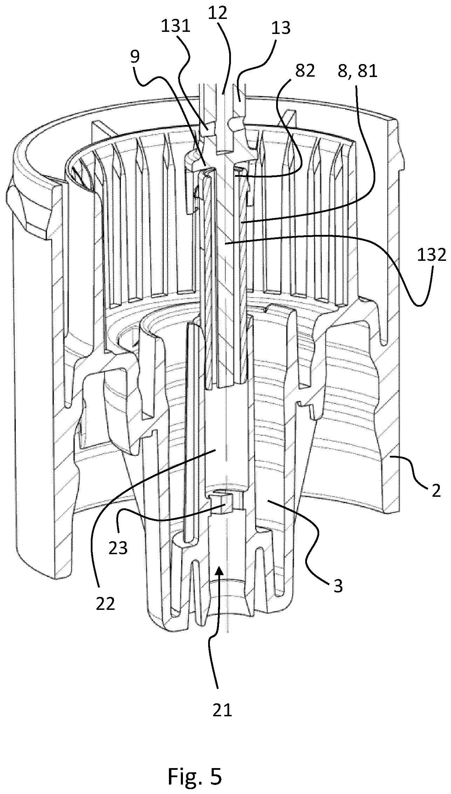

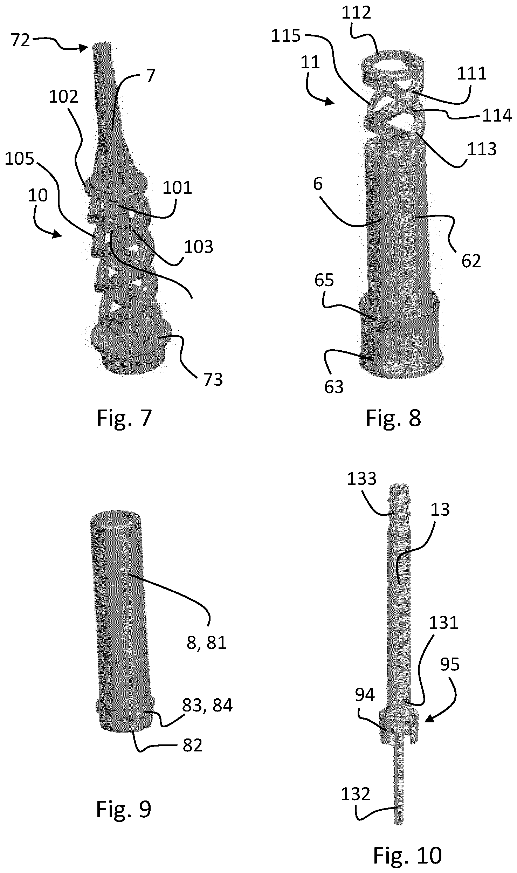

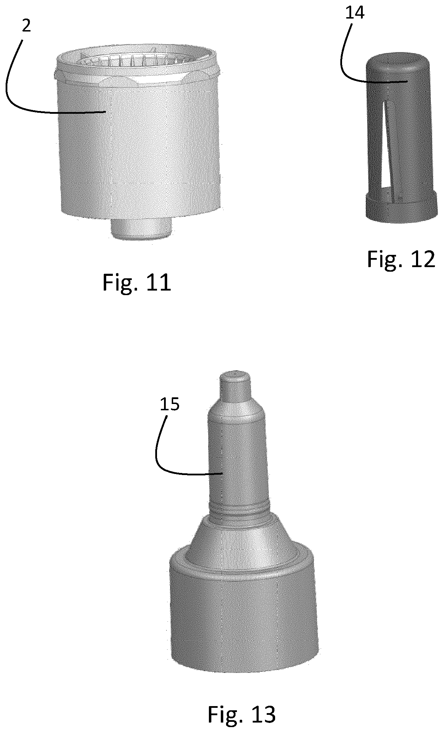

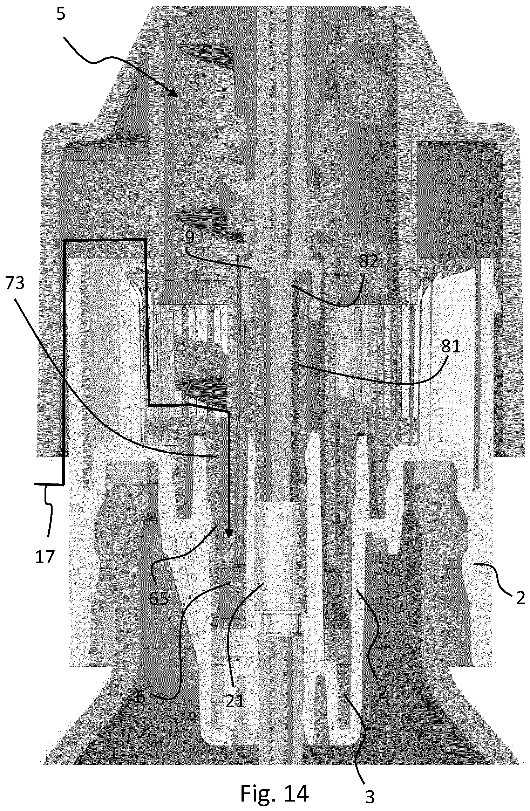

With reference to Figs. 1 to 13, an exemplary design of a manual pump 1 according to the embodiment is described. With reference to Figs. 14 to 19, an exemplary use of the manual pump 1 is described. In some figures, reference signs have been omitted for simplicity reasons.

The manual pump 1, such as a manual multi-dose spray pump (e.g., used as a nasal pump or pump for dispensing a fluid into a nose), is provided for pumping a fluid, such as a gas or a liquid. The pump 1 may be suitable for any container or container body, such as a bottle. In particular, the pump 1 is suitable for a fluid dispenser, such as a fluid dispenser for dispensing a fluid for intranasal administration. The manual pump 1 may suitable for dispensing a fluid in an aerosolized form, such as in the form of a spray.

The pump 1 comprises a pump body 2 delimiting a pump chamber 3 (such as a metering or compression chamber) and having an inlet 21 connectable, or connected, to a reservoir 4. The inlet may be connectable to a (dip) tube 20 extending into the reservoir 4. The reservoir 4 may be provided by a container body 200. The reservoir 4 may contain the fluid. The pump body 2 may comprise a duct 22 that may extend axially and/or may comprise the inlet 21. The pump body 2 may comprise an opening 25 that can be fluidly connected to the reservoir 4, wherein the pump body 2 delimits at least part of a flow path 17 that has a first end connected to the opening 25 and a second end connected to an outside 18 (the environment of the pump 1, such as the atmosphere). The pump body 2 may be designed as a closure for a container or container body. The pump body 2 may comprise fastening means 24, such as a thread and/or a snap fit, for connecting the pump to the container body 200.

The pump 1 comprises a piston assembly 5 comprising a piston 6 and a piston rod 7, such as a piston stem. The piston 6 may comprise a hollow body that delimits the pump chamber 3. The piston 7, or the hollow body, may comprise one or more walls, such as a (e.g., radially extending) top wall 61 and an (e.g., axially extending) sidewall 62. The piston 6 may comprise a sealing element 63, such as a sealing lip, arranged to contact an inner side of the pump body 2. The piston 6 may comprise an outlet valve section 64 configured to move between an open position and a closed position. The open position allows a fluid flow from the pump chamber 3 into an outlet channel 12, which outlet channel 12 may be provided at least partly in the piston rod 6. The closed position prevents a fluid flow from the pump chamber 3 into the outlet channel 12. The outlet valve section 64 is configured to move into the open position when a pressure inside the pump chamber 3 exceeds a defined pressure. For example, the outlet valve section 64 may be provided at least partly by the top wall 61 and/or may delimit the pump chamber 3, e.g. at an upper side thereof.

The piston rod 7 may comprise, or contain, at least part of the outlet channel 12. The piston rod 7 may comprise a dispensing opening 72, e.g. arranged on an upper section or on the tip of the piston rod 7. The dispensing opening 72 may be fluidly connected to the outlet channel 12. The dispensing opening 72 may be configured to generate an aerosol from a fluid. For example, the dispensing opening 72 may comprise, or may be connected to, an atomizer, such as a swirl atomizer (Fig. 2A). The swirl atomizer may comprise a (open) swirl chamber through which a fluid, such as a liquid, flowing out of the outlet channel 12 can pass and/or may comprise an orifice or nozzle, through which the fluid can exit. As the fluid exits via the orifice or nozzle at high speed, the centrifugal forces cause the fluid to spread out and disintegrate into fine droplets, thus achieving atomization.

The piston assembly 5 may comprises a piston connector 13. The piston connector 13 may extend axially and/or may be in the form of a plug. The piston connector 13 is attached, preferably by a snap fit 133, to the piston rod 7. At least part of the piston connector 13 may be arranged or received in the piston rod 6. For example, the piston connector 13 engages, e.g. by one or more snap elements or any other fastening means (such as a thread), in a corresponding section of the piston rod 7. The corresponding section may comprise corresponding snap elements or a thread. The piston connector 13 may be arranged to connect the piston 6 to the piston rod 7, or vice versa. The outlet channel 12 may be arranged (or may extend) at least partly in the piston connector 13. For example, a first (downstream) part of the outlet channel 12 extends in the piston connector 13 and a second (upstream) part of the outlet channel 12 extends in the piston rod 7.

The piston connector 13 may comprise an inlet 131 through which a fluid can flow from the outlet valve section 64 into the outlet channel 12. The outlet valve section 64 may be axially moveable relative to the piston connector 13 such that a pressure inside of the pump chamber 3 acting directly or indirectly on the outlet valve section 64 can move the outlet valve section 64 relative to the piston connector 13. Being in the closed position, the outlet valve section 64 may be supported on the piston connector 13, wherein in the open position, the outlet valve section 64 and the piston connector 13 may delimit an opening 641 connecting the pump chamber 3 to the outlet channel 12. In this position, a fluid may flow from the pump chamber 3 via the opening 641 into a space delimited by the piston 6 and piston connector 13, from which space the fluid can flow via the inlet 131 into the outlet channel 12.

At least part of the piston connector 13 may be arranged in a space delimited by the piston 6. The piston connector 13 may comprise an axial, preferably central protrusion 132 extending axially. The protrusion 132 may extend into the duct 22.

The pump 1 further comprises a valve 8, such as a check valve, a valve providing the function of a check valve (or one-way valve) or a valve part of a check valve system. The valve 8 comprises an axially moveable valve body 81 with an opening 82 for conducting a fluid from the inlet 21, and thus, e.g., from the reservoir 4, into the pump chamber **3.** The opening 82 extends through the valve body 81, such as to form an inlet at a first, lower end of the valve body 81 and an outlet at a second, upper end of the valve body 81. The opening 82 may be a through hole. The valve body 81 may be axially guided by the pump body 2, such as by the duct 22, and/or may be connected to the pump body 2 by a frictional connection so that the valve body 81 is movable with a mechanical resistance defined by the frictional connection. The pump body 2 may comprise a stopper 23, such as in the form of a protrusion (e.g., a so-called "bridge"), arranged to limit a movement of the valve body 81 along an axial downward direction. The stopper 23 particularly simplifies an assembly of the valve body 81. The stopper 23 may be arranged at least partly in the duct 22. The stopper 23 may also serve as a (mechanical) stop for the tube 20. The valve body 81 may be arranged such that the protrusion 132 extends into the opening 82 of the valve body 81, e.g. for guiding the valve body 81. The valve body 81 may have a tubular shape and/or is a sleeve.

The piston assembly 5 comprises a closing section 9 attached, directly or indirectly, to the piston rod 7. The piston connector 13 may comprise the closing section 9, so that the closing section 9 may be indirectly, via the piston connector 13, attached to the piston rod 7. The closing section 9 may be flat and/or may be annular, such as an annular surface. The closing section 9 may be arranged opposite and/or above the opening 82 of the valve body 81.

The pump 3 comprises one or more resilient elements 10, 11 arranged to interact with the piston assembly 5, wherein the one or more resilient elements 10, 11 are integrally formed with the piston 6 and/or piston rod 7. The one or more resilient elements 10, 11 may be provided together or without the valve body 81 and the closing section 9. The one or more resilient elements 10, 11 may comprise a return resilient element 10 arranged to move at least the piston rod 7 axially upwards when an actuation pressure to move the piston rod 7 axially downwards is removed. The return resilient element 10 may be integrally formed with the piston rod 7. The one or more resilient elements 10, 11 may comprise a (outlet) valve resilient element 11 to bias (or pre-compress or pre-tension) the outlet valve section 64 into the closed position. The valve resilient element 11 may be, at a first end, integrally formed with the piston 6, such as with the outlet valve section 62. At a second end, the valve resilient element 11 may be connected to the piston rod 7. In particular, the valve resilient element 11 may be arranged between a section of the piston 6 and a section of the piston rod 7. The one or more resilient elements 10, 11 and preferably the piston assembly 5 may be arranged coaxially.

The return resilient element 10 may be a spring, e.g. a so-called "Hana"-spring. The return resilient element 10 comprise a top plate 102, such as top ring plate having a hole formed at the center thereof, first and second spring members 101, 103 and a pair of connection spring members 104, 105. The first spring member 101 is erectly extended downward from one side undersurface of the top plate 102 while rotating in a clockwise direction. The second spring member 103 is erectly extended downward from the other side undersurface of the top plate 102 which is a position symmetrical to one side undersurface thereof, while rotating in the same direction as the first spring member 101. The pair of connection spring members 104, 105 is erectly extended downward from two center surfaces between one side undersurface and the other side undersurface of the top plate 102, while rotating in the opposite direction (in a counterclockwise direction) to the first and second spring members 101, 103, in such a manner as to be connected alternately to tops and undersides of the first spring member 101 and the second spring member 103. For example, the spring may be according to the cylindrical spring (260) in EP 3 854 486 B1.

The valve resilient element 11 may be a spring, e.g. a so-called "Hana"-spring. The valve resilient element 11 comprise a top plate 112, such as top ring plate having a hole formed at the center thereof, first and second spring members 111, 113 and a pair of connection spring members 114, 115. The first spring member 111 is erectly extended downward from one side undersurface of the top plate 112 while rotating in a clockwise direction. The second spring member 113 is erectly extended downward from the other side undersurface of the top plate 112 which is a position symmetrical to one side undersurface thereof, while rotating in the same direction as the first spring member 111. The pair of connection spring members 114, 115 is erectly extended downward from two center surfaces between one side undersurface and the other side undersurface of the top plate 112, while rotating in the opposite direction (in a counterclockwise direction) to the first and second spring members 111, 113, in such a manner as to be connected alternately to tops and undersides of the first spring member 111 and the second spring member 113. For example, the spring may be according to the cylindrical spring (260) in EP 3 854 486 B1.

The pump 1 may comprise an attachment section 73 for attaching the piston assembly 5 to the pump body 2, e.g. by a snap fit. The attachment section 73 may comprise a plate 731, such as a ring plate, wherein one or more (e.g., annular) attachment sub-sections 732, by which the attachment section 73 can be attached to the pump body 2, may extend (e.g., axially, or transversely, such as perpendicularly, with respect to the plate 731) from the plate 731. The one or more attachment sub-sections 732 may comprise one or more snap elements. The attachment section 73 may remain stationary as the piston assembly 5 moves axially. The attachment section 73 may be integrally formed with the piston assembly 5, such as with the piston rod 7. The return resilient element 10 may be integrally formed with the attachment section 73. The attachment section 72 may be arranged to delimit at least part of the flow path 17. For example, at least part of the flow path 17 extends between a section of the pump body 2 and a section of the attachment section 73. Preferably, the attachment section 73 comprises one or more (e.g., axially extending) ribs 19 that contact the piston 6 (e.g. on an outer side thereof) and/or delimit the flow path 17. The attachment section 73 may be annular and/or may comprise an opening 733, wherein the piston 6 and/or the piston connector 13 may extend through the opening 733.

The pump 1, or an assembly comprising the pump 1, may comprise a cap 14 (over cap) arranged to cover the dispensing opening 72 provided in the piston rod 7.

The pump 1 may comprise a manually operable actuator 15 arranged to enclose at least part of the piston assembly 5 and at least part of the pump body 2. Preferably, the actuator 15 is axially moveable relative to the pump body 2. The actuator 15 may be arranged to delimit at least part of the flow path 17. In particular, at least part of the flow path may be arranged between a section of the actuator 15 and a section of the pump body 2. The actuator 15 may be attached to the piston rod 7, e.g. by a snap fit or another fastening means such as a threaded connection. The actuator 15 may comprise an opening 151 that is fluidly connected to the dispensing opening 72 or the aerosolizer, so that, for example, a fluid, e.g. in an aerosolized form, can exit via the opening 151. The actuator 15 may be stepped and/or may comprise a (e.g., radially extending) surface 152 for actuation, e.g. by a human finger.

The pump 1 may comprise a seal 16 arranged on the pump body 2 and arranged to contact the container body 200. The seal 16 may be arranged on an underside of the pump body 2. When the pump body 2 is attached to the container body 200, the seal 2 is clamped between the pump body 2 and the container body 200, e.g. a neck thereof. The seal 16 may be a sealing element such as gasket or a sealing ring.

The valve body 81 is not limited to a specific material. Preferably, the valve body 81 is made of a plastic material, such as polypropylene (PP). The one or more resilient elements 10, 11 is/are not limited to a specific material. Preferably, the one or more resilient elements 10, 11 is/are made of a plastic material, such as polypropylene (PP). Preferably, the pump body 2, the piston assembly 5 and the valve body 81 are all made of the same material, such as a plastic material, preferably PP. Preferably, the pump body 2, the piston assembly 5 and the one or more springs 10, 11 are all made of the same material, such as a plastic material, preferably PP.

With reference to Figs. 14 to 19, an exemplary operation of the pump 1 is described.

Fig. 14 shows the pump assembly 5 in an upper, or initial or starting, position. The opening 82 of the valve body 81 is distanced from the closing section 9, so that the inlet 21 is fluidly connected with the pump chamber 3 via the opening 82. The flow path 17 is blocked by the piston 6, such as by the wedge-shaped element 65 of the piston 6 inserted between a section of the pump body 2 and a section of the attachment section 73. From the shown position, the piston assembly 5 can move axially downwards to set a dose of fluid in the pump chamber 3 under pressure.

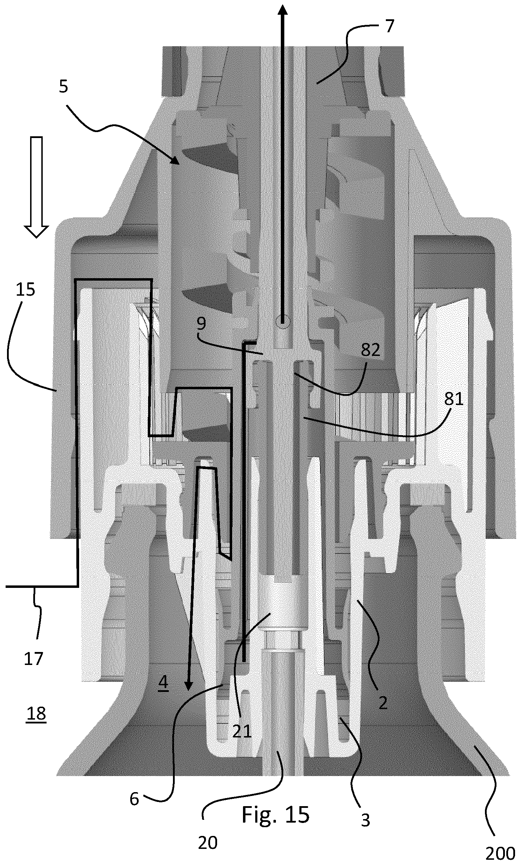

Fig. 15 shows a state in which the piston assembly 5 moves, e.g. by a user's actuation pressing the actuator 15, from the position shown in Fig. 14 axially downwards, in particular towards the container body 200. As the piston rod 7 moves axially downwards, the closing section 9 comes into contact, or hits, the valve body 81 (i.e. its outlet or upper end) and closes the opening 82. Thus, a flow of fluid from the pump chamber 3 into the inlet 21, and thus into the tube 20, is prevented. In other words, backflow via the inlet 21 into the (dip-)tube 20 may be prevented. Pressure builds up in the pump chamber 3 due to the piston 6 moving downwardly, the dose of fluid in the pump chamber 3 is set under pressure. At the same time, the piston 6 is arranged in a lower position, in which the flow path 17 is not blocked by the piston 6, so that a gas, such as air, can flow from the outside 18 into the inside 4 of the container body 2.

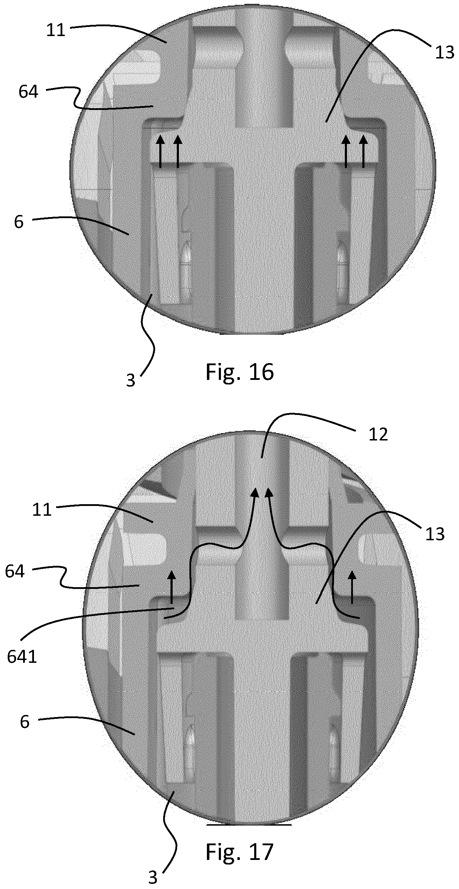

In a first phase during the motion axially downwards, the pressure inside of the pump chamber 3 is below a (defined) pressure required to move the outlet valve section 64 into the open position. A state of this phase is shown in Fig. 16. In particular, the pressure inside of the chamber 3 is too low to move the outlet valve section 64 against the force exerted by the resilient element 11. The piston connector 13 is pressed against the outlet valve section 64, as indicated by the vertical arrows in Fig. 16. The outlet valve section 64 remains in its closed position. The reason why the outlet valve section 64 remains in this phase in the closed position is to achieve an advantageous dispensing, e.g. via the dispensing opening 72, from the pump **1.** In particular, the atomizer, which may be a swirl atomizer, needs a minimum pressure to create enough speed which in turn is needed to break up the particles. Thus, by the outlet valve section 64 this minimum pressure can be ensured.

In a second phase during the motion axially downwards, in which second phase the piston 6 is moved further downwards and the volume of the pump chamber 3 is thus further reduced, the pressure inside of the pump chamber 3 exceeds the defined pressure, thereby moving the outlet valve section 64 into the open position. A state of this phase is shown in Fig. 17. In particular, the pressure inside of the chamber 3 is such that a force greater than the force exerted by the resilient element 11 is effected, whereby the outlet valve section 64 moves (relative to the piston connector 13) axially upwards, as indicated by the vertical arrows shown in Fig. 16; in other words, the built-up pressure is high enough to move the resilient element 11 (e.g., in the form of a spring), creating the opening 641 between the piston connector 13 and the piston 6. The outlet valve section 64 is in its open position and thus allows a fluid flow from the pump chamber 3 into the outlet channel 12. Fluid flows in the outlet channel 12 and further downstream to be dispensed via the dispensing opening 72 from the pump 1, e.g. in an aerosolized form or as a spray.

As the piston assembly 5 moves axially downwards, the opening 82 is closed by the closing section 9 in such a way that a seal is provided between the closing section 9 and the valve body 81. This seal is preferably adapted to withstand a pressure at least equal to the defined pressure required to move the outlet valve section 64 into its open position. In particular, the friction between the valve body 61 and the pump body 2 may be such that the closing section 9 is pressed against (an upper part of) the valve body 81 to provide the seal, as the closing section 9 moves axially downwards.

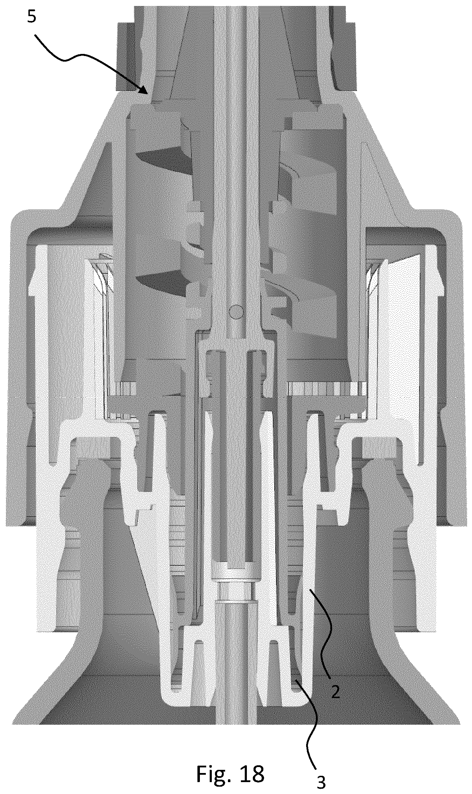

The down stroke, i.e. the motion of the piston assembly 5 axially downwards, ends with the (lower) position shown in Fig. 18. In this position, substantially all of the dose of the fluid is dispensed from the pump chamber 3. A new dose of fluid can be sucked into the pump chamber 3. From the position shown in Fig. 18, the piston assembly 5 can move, or return, axially upwards, to its initial position, in order to suck a dose of fluid into the pump chamber 3.

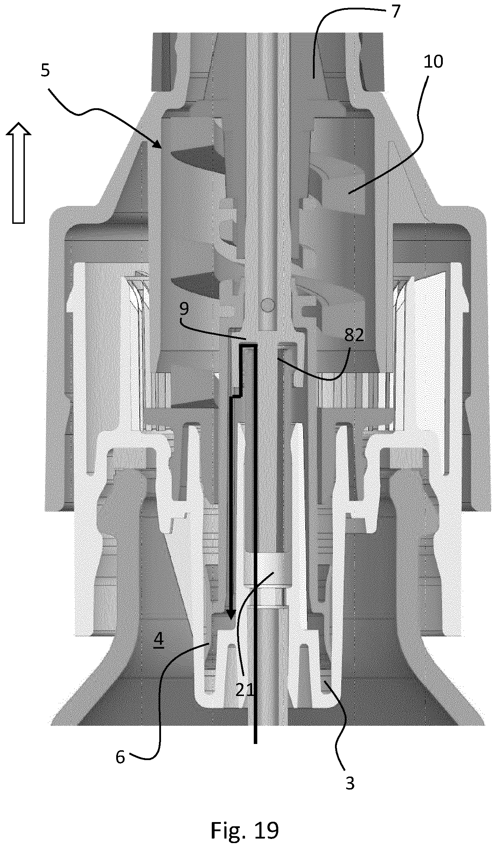

Fig. 19 shows a state in which the piston assembly 5 moves from the position shown in Fig. 18 axially upwards, e.g. by the (action of the) resilient element 10 when an actuation pressure to move the piston rod 7 axially downwards is removed. As the piston rod 7 moves axially upwards, the closing section 9 moves away from the valve body 81 and becomes distanced from, and thus exposes, the opening 82. Thus, a flow of fluid from the inlet 21, and thus from the reservoir 4 connected to the inlet 21, into the pump chamber 3 is allowed. Pressure decreases in the pump chamber 3 due to the piston 6 moving axially upward, thereby creating an underpressure in the pump chamber 3. This reduced pressure (e.g., a pressure below atmosphere) effects a flow of fluid from the inlet 21 via the opening 82 into the pump chamber 3, so that a (fresh dose of) fluid is sucked or pulled into the pump chamber 3, which is thus filled with the fluid. This is indicated by the arrow going up and down in Fig. 19. Thereby, the fluid is removed from the reservoir 4 (pressure reduction in the reservoir 4). At the same time, the piston 6 is in a lower position, in which the flow path 17 is not blocked by the piston 6, so that a gas, such as air, can flow from the outside 18 into the reservoir 4 of the container body 2 to replace the space from the fluid removed from the reservoir 4.

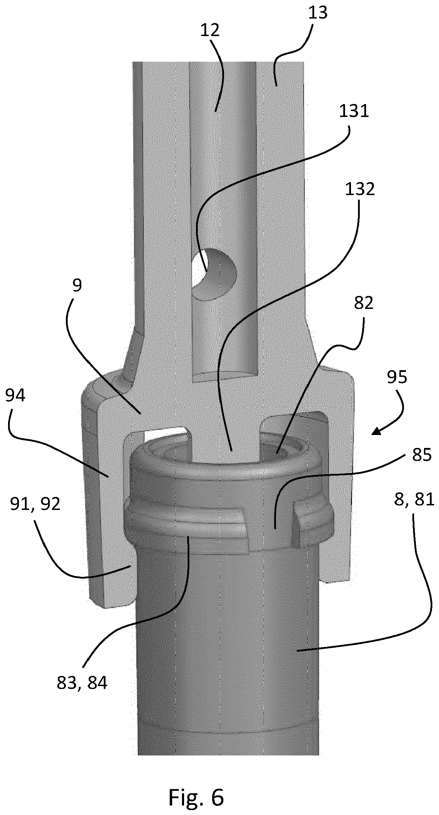

As the piston rod 7 moves axially upwards, the piston rod 7 connects to the valve body 81 via a connection 83, 91 (Fig. 6 and Figs. 9 and 10) and, as soon as the connection 83, 91 is provided, the piston rod 7 and the valve body 81 move together axially upwards. In other words, when the connection 83, 91 is provided, the piston rod 7 moving axially upwards carries the valve body 81 axially upwards. Thus, at least during part of the motion of the piston rod 7 axially upwards, the closing section 9 is distanced from the opening 82 by a fixed value. The connection 83, 91 may comprises a first engagement section 91 attached, directly or indirectly, e.g. via the piston connector 13, to the piston rod 7 and a second engagement section 83 attached to the valve body 81. The first engagement section 91 may be attached to the piston connector 13, such as to the axial wall 94 or the cage 95. The first engagement section 91 may extend radially, such as radially inwards. The second engagement section 83 may be attached to an outer or inner side of the valve body 81. The second engagement section 83 may extend radially, such as radially outwards. Each of the first engagement section 91 and/or the second engagement section 83 may be provided by a respective protrusion 92, 84, such as a snapping protrusion.

The valve body 81 may be connected to the piston rod 7, in particular to the piston connector 13, with axial play, which means that the valve body 81 and the piston rod 7 may move axially relative to one another. Preferably, the axial play is in a range of 0.1-1mm.

During each of the down stroke and up stroke described above, the piston assembly 5, in particular the piston rod 7, may move along a distance in a range of 4-8mm, preferably 5-7mm.

It should be clear to a skilled person that the embodiment shown in the figures is only a preferred embodiment, but that, however, also other designs of a pump 1 can be used.

## Claims

1. A manual pump (1) for a fluid dispenser, such as a fluid dispenser for dispensing a fluid for intranasal administration, the pump (1) comprising:
- a pump body (2) delimiting a pump chamber (3) and having an inlet (21) connectable to a reservoir (4),
- a piston assembly (5) comprising a piston (6) and a piston rod (7), wherein the piston assembly (5) is arranged to move axially downwards to set a dose of fluid in the pump chamber (3) under pressure and arranged to move axially upwards to suck a fluid from the reservoir (4) into the pump chamber (3) via the inlet (21),
- a valve (8) arranged to prevent a flow of the fluid from the pump chamber (3) into the inlet (21) as the piston assembly (5) moves axially downwards and to allow a flow of the fluid from the inlet (21) into the pump chamber (3) as the piston assembly (5) moves axially upwards,
wherein:
a) the piston assembly (5) comprises a closing section (9) attached to the piston rod (7), and the valve (8) comprises an axially moveable valve body (81) with an opening (82) for conducting a fluid from the inlet (21) into the pump chamber (3), wherein the closing section (9) and the valve body (81) are arranged such that
- the opening (82) is closed by the closing section (9) as the piston rod (7) moves axially downwards, so that a flow of fluid from the pump chamber (3) into the inlet (21) is prevented, and
- the opening (82) is distanced from the closing section (9) as the piston rod (7) moves axially upwards, so that a flow of fluid from the inlet (21) into the pump chamber (3) is allowed; and/or
b) the pump (1) comprises one or more resilient elements (10, 11) arranged to interact with the piston assembly (5), wherein the one or more resilient elements (10, 11) are integrally formed with the piston (6) and/or piston rod (7).

2. The pump (1) according to claim 1, wherein the valve body (81) can be connected via a connection (83, 91) to the piston rod (7) such that when the piston rod (7) moves axially upwards the valve body (81) moves axially upwards as well.

3. The pump (1) according to claim 2, wherein the connection (83, 91) comprises a first engagement section (91) attached to the piston rod (7) and a second engagement section (83) attached to the valve body (81), wherein, preferably, each of the first engagement section (91) and/or the second engagement section (83) is provided by a respective protrusion (92, 84), such as a snapping protrusion.

4. The pump (1) according to any of the preceding claims, wherein the valve body (81) is connected to the piston rod (7) with axial play, wherein the axial play is preferably in a range of 0.1-1mm.

5. The pump (1) according to any of the preceding claims, wherein the valve body (81) is axially guided by the pump body (2), such as by a duct (22) of the pump body (2), wherein the duct (22) preferably comprises the inlet (21), and/or wherein the valve body (81) is connected to the pump body (2) by a frictional connection.

6. The pump (1) according to any of the preceding claims, wherein the pump body (2) comprises a stopper (23), such as in the form of a protrusion, arranged to limit a movement of the valve body (81) along an axial downward direction.

7. The pump (1) according to any of the preceding claims, wherein the piston (6) comprises an outlet valve section (64) configured to move between an open position that allows a fluid flow from the pump chamber (3) into an outlet channel (12), e.g. provided at least partly in the piston rod (6), and a closed position that prevents a fluid flow from the pump chamber (3) into the outlet channel (12), wherein the outlet valve section (64) is configured to move into the open position when a pressure inside the pump chamber (3) exceeds a defined pressure.

8. The pump (1) according to any of the preceding claims, wherein, when the opening (82) is closed by the closing section (9), a seal is provided between the closing section (9) and the valve body (81), wherein the seal is preferably adapted to withstand a pressure at least equal to the defined pressure.

9. The pump (1) according to any of the preceding claims, wherein the piston assembly (5) comprises a piston connector (13), such as a in the form of a plug, attached, preferably by a snap fit (133), to the piston rod (6) and comprising the closing section (9).

10. The pump (1) according to claim 9, comprising an outlet channel (12) that is at least partly arranged in the piston connector (13), wherein the piston connector (13) preferably comprises an inlet (131) through which a fluid can flow from the outlet valve section (64) into the outlet channel (12).

11. The pump (1) according to any of the preceding claims, wherein the one or more resilient elements (10, 11) comprise:
- a return resilient element (10) arranged to move at least the piston rod (7) axially upwards when an actuation pressure to move the piston rod (7) axially downwards is removed, wherein the return resilient element (10) is preferably integrally formed with the piston rod (7), and/or
- a valve resilient element (11) arranged to bias an outlet valve section (64), such as the outlet valve section (64), into a closed position, wherein the valve resilient element (11) is preferably integrally formed with the piston (6).

12. The pump (1) according to any of the preceding claims, wherein the pump body (2) comprises an opening (25) that can be fluidly connected to a reservoir (4) of a container body (200), wherein the pump body (2) delimits at least part of a flow path (17) that has a first end connected to the opening (25) and a second end connected to an outside (18), wherein the piston (6) is configured to move from an upper position, in which the flow path (17) is blocked by the piston (6), preferably by a wedge-shaped element (65) of the piston (6), axially downwards into a lower position, in which the flow path (17) is not blocked by the piston (6), so that a gas, such as air, can flow from the outside (18) into the reservoir (4) of the container body (2).

13. The pump (1) according to any of the preceding claims, wherein the valve body (81) and/or the one or more resilient elements (10, 11) is/are made of a plastic material, such as polypropylene (PP).

14. A container (1000) comprising a container body (200) and a manual pump (1) according to any of the preceding claims.

15. A method for manually pumping a fluid from a reservoir, comprising the steps of:
- moving, in a down stroke, a piston assembly (5) comprising a piston (6) and a piston rod (7) axially downwards to set a dose of fluid in a pump chamber (3) under pressure, wherein during at least part of the down stroke an opening (82) of a valve body (81) is closed by a closing section (9) attached to the piston rod (7) so that a flow of fluid from the pump chamber (3) via the opening (82) into an inlet (21) connected to the valve body (81) is prevented, and
- moving, in an up stroke, the piston assembly (5) axially upwards, wherein during at least part of the up stroke the opening (82) is distanced from the closing section (9) so that a flow of fluid from the inlet (21) via the opening (82) into the pump chamber (3) is allowed.
